# EUROPEAN PATENT APPLICATION

(11) **EP 2 184 044 A1**
(43) Date of publication of application: **12.05.2010**
(21) Application number: 08168518.2
(22) Date of filing: 06.11.2008
(51) Int. Cl.: A61F 13/20, A61F 13/22

(54) **Tampon with anti-leakage element, method and apparatus for producing the same**

(71) Applicant: Ontex Hygieneartikel Deutschland GmbH, 02692 Grosspostwitz (DE)
(72) Inventor: Smet, Steven, 9240 Zele (BE)
(74) Representative: Brants, Johan P.E.

(57) **Abstract**

The present invention relates to tampons, in particular to tampons for insertion into body orifices or cavities, such as catamenial tampons. In particular, the invention concerns a tampon comprising an elongated absorbent core having an upper insertion end, a lower withdrawal end, **characterised in that** said tampon is provided with a diameter-restraining element at the withdrawal end. In another aspect, the invention relates to a method for manufacturing such tampons and to an apparatus configured to produce such tampons.

## Description

### FIELD OF THE INVENTION

The present invention relates to tampons, in particular to tampons for insertion into body orifices or cavities, such as catamenial tampons. In another aspect, the invention relates to a method for manufacturing such tampons and to an apparatus configured to produce such tampons.

### BACKGROUND OF THE INVENTION

Tampons are used for hygienic and/or medical purposes. For example, catamenial tampons are commonly used by women to absorb menstrual fluid within the vaginal cavity. Conventional tampons include an absorbent core comprising an absorbent material e.g. hydrophilic fibres or foam. Tampons have an elongated shape and can be manufactured using a process including folding, compressing and/or moulding steps.

Tampons absorb part of the liquid and store it in their absorbing core, thus causing their own expansion. When tampons are used in a narrow channel, such as the vagina, their expansion leads to the formation of a plug. This can prevent additional upstreaming liquid from leaving the channel. However, when a tampon is completely saturated, it starts leaking at its withdrawal end and/or withdrawal cord. Thus, there is a need for new tampons avoiding withdrawal end leakage.

In order to combine a minimised risk for e.g. Toxic Shock Syndrome with an optimal protection against leakage and best wearing comfort, it is important to select the proper size of tampon. The 'optimal' size is likely to vary according to the time within the menstrual period. Unfortunately, tampons look very similar once they have been removed from their consumer package. This may result in unwilling and unknowing use of the wrong size of tampons.

Finally, tampons display a significant change of dimensions between their original and their swollen state. Due to this change in size and shape, they are sometimes difficult to remove. Swollen state tampons create strains or frictions when they are withdrawn from the orifice and increase the risk of fibre loss. It is also known in the art that tampons can cause frictions against the sensitive tissue of the vaginal channel. It is thus important to provide tampons which avoid above-cited inconveniences.

Consequently, there is a need to further improve tampons. More in particular, there is the need to improve their performance in the frequent situations when their absorbent core is completely saturated and starts leaking.

### SUMMARY OF THE INVENTION

The aspects of the present invention address at least some, e.g., one or more, of the above discussed needs of the art (i.e. withdrawal end leakage, product identification and easy removal). In particular, the inventors surprisingly found that providing a tampon comprising a diameter-restraining element with a specific configuration at the withdrawal end can greatly improve the ability of such tampons to cope with withdrawal end leakage or frictions of the vaginal channel.

As a further advantage, the inventors realised that a tampon with the above configuration displays a surface reducing friction compared to the traditional tampons. The tampon according to the invention is thus contemplated to be more pleasant to consumers (e.g., causing smoother and more comfortable sensation upon withdrawal of the tampon). Moreover, a tampon with the above configuration, i.e. with a diameter restraining element according to the invention, can act as an anti-leakage guard to avoid leakage at the withdrawal end of the tampon.

Accordingly, in an aspect, the invention provides a tampon comprising an elongated absorbent core having an insertion end, a withdrawal end, **characterised in that** said tampon is provided with a diameter-restraining element at the withdrawal end. In a preferred embodiment, after use, the diameter of the withdrawal end may be lower than the average diameter of the tampon. When provided at the withdrawal end, the diameter restraining element allows to reduce strains or frictions when the tampon is withdrawn from the orifice and to reduce the risk of leaving particles in the vagina.

In a preferred embodiment, the diameter-restraining element may be hydrophobic or liquid-impervious. Such feature allows the diameter-restraining element to be an anti-leakage element in addition to a restraining element. Hence, the user will be advantageously secured with a tampon having a hydrophobic or liquid-impervious diameter-restraining element. Indeed, this latter provides smooth, non-drying feeling and preferably reduced friction upon removal of the tampon from a body cavity, *e*.*g*., a vaginal cavity.

It is finally another advantage of the present invention to avoid leakage by providing an identification element on the outer surface of the tampon. The identification element allows the selection of the proper size of tampon. Hence, the tampon will closely fit to the vagina and will improve user's comfort.

In another aspect, the invention relates to a method of manufacturing tampons provided with a diameter-restraining element, to the use of such tampons and to an apparatus configured to produce such tampons.
These and further aspects and preferred embodiments of the invention are described in the following sections and in the appended claims.

### SHORT DESCRIPTION OF THE FIGURES

**FIG. 1** represents a tampon according to an embodiment of the invention.
**FIG. 2** represents one step in a process for producing a tampon according to an embodiment of the invention.
**FIG. 3** represents the folding step of the diameter-restraining element around the elongated absorbent core.
**FIG. 4** represents the manufacturing of the tampon blank.
**FIG. 5** represents the tampon blank prior to its insertion in the tampon pressing section of a tampon machine.
**FIG. 6** represents the finished product.
**FIG. 7** represents the tampon according to an embodiment of the invention after use.
**FIG. 8** represents a tampon provided with two identification elements.
**FIG. 9** represents a tampon, after use, provided with a diameter-restraining element made of a plurality of discontinuous strips.

### DETAILED DESCRIPTION

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps.

The recitation of numerical ranges by endpoints includes all numbers and fractions subsumed within that range, as well as the recited endpoints.

The term "about" as used herein when referring to a measurable value such as a parameter, an amount, and the like, is meant to encompass variations of +/-20% or less, preferably +/-10% or less, more preferably +/-5% or less, even more preferably +/-1 % or less, and still more preferably +/-0.1 % or less of and from the specified value, insofar such variations are appropriate to perform in the disclosed invention.

Unless otherwise defined, all terms used in the disclosure of the invention, including technical and scientific terms, have the meaning as commonly understood by one of ordinary skill in the art to which this invention belongs. By means of further guidance, term definitions are included to better appreciate the teaching of the present invention.

The term "tampon" broadly encompasses any type of absorbent structure used for inserting into openings, orifices, cavities or canals with the purpose of absorbing liquid or moisture potentially present therein or discharged there from. For example, a tampon may be configured and intended for inserting into a body cavity or canal of an animal. Preferably, the animal is mammal, more preferably human. For example, a tampon may be inserted into the vaginal canal, urinal canal, rectum, ear canal, nasal canal or throat. Alternatively, a tampon can also be used in such body orifices to deliver substances such as drugs, microflora or moisture. In another example, a tampon may be configured to be inserted within industrial installations, equipments or appliances, such as for instance tubing or piping. A preferred tampon type may be a catamenial tampon, configured for insertion into the vaginal canal of human females, for absorbing and preventing the leakage of menstrual fluid.

The present invention concerns advancements to the surface of tampons, whereby the overall characteristics and performance of tampons are improved. Therefore, the skilled reader shall appreciate that the teachings of the present invention are generally applicable to a wide variety of tampons, including various types and forms of catamenial tampons. The description of tampons, especially of catamenial tampons, serves to exemplify, but not to limit, the types of tampons to which the invention can preferably apply.

The teachings of the invention may be applicable to self-sustaining shape tampons, deformable or fluid-permeable bag tampons. Self-sustaining shape tampons are presently in widespread use, and non-limiting, exemplary disclosures thereof include, *e*.*g*., GB 394,571, GB 490,024, DE 3,934,153 C2 and US 5,911,712 incorporated by reference herein. Exemplary deformable, fluid-permeable bag tampons are disclosed in, *e*.*g*., US 3,815,601 and US 4,278,088, incorporated by reference herein.

In one embodiment of the invention, a tampon may comprise an elongated absorbent core having an insertion end, a withdrawal end, **characterised in that** said tampon is provided with a diameter-restraining element at the withdrawal end.

The insertion end corresponds to the end which leads the insertion of the tampon into a body cavity. The withdrawal end, opposite to the insertion end, guides the withdrawal of the tampon from the body cavity. The elongated absorbent core of the tampon comprises an absorbent material which can be a hydrophilic material. For example, the absorbent material may be rayon, cotton, wood pulp, comminute wood pulp, creped cellulose wadding, tissue wraps and laminates, peat moss, and chemically stiffened, modified, or cross-linked cellulosic fibres; polymeric materials (*e*.*g*. polyester fibres, polyurethane foam, absorbent sponges, super-absorbent polymers, absorbent gelling materials), synthetic fibres, wood pulp or any equivalent materials, or mixtures thereof. The tampon may preferably comprise a withdrawal cord attached to the withdrawal end to facilitate withdrawal of the tampon after use. The withdrawal cord is preferably flexible, hydrophobic and long enough to protrude from the body cavity. The withdrawal cord is also of sufficient tensile strength to resist breaking during removal of the tampon. A withdrawal cord may be, *e*.*g*., a single cord, a tape, or a plurality of strings. Exemplary, non-limiting materials may be cotton, rayon, hydrophobic cotton, hydrophobic polyester or a mixture thereof.

A preferred tampon according to an embodiment of the invention may have a cylindrical shape. The term "cylindrical" does not refer necessarily to a right circular cylinder, but rather to a shape that can be approximated to a geometrical cylinder or a derivative thereof. Hence, the term encompasses a typical cylinder form, a truncated cone form (a frustoconical shape), a barrel form (pineapple shape), oblate or partially flattened cylinder forms, curved cylinder forms, cylindrical forms with varying cross-sectional areas and the like. It shall be appreciated that while a tampon may have an overall cylindrical shape, its surface may include profiles such as variably shaped grooves or ribs. The term "cylindrical" refers to the usual shapes of tampons, especially catamenial tampons, as well-known in the art.

The tampon of the invention may comprise grooves and/or ribs. Preferably, the grooves may be longitudinal, i.e., extending along at least a portion of the length of the tampon. Preferably, the longitudinal grooves may be generally parallel to the central longitudinal axis of the tampon. Preferably, the longitudinal grooves may extend along at least 50%, preferably at least 70%, more preferably at least 90%, or even more preferably about 100% of the axial length of the elongated surface of the tampon. A groove may extend over various portions of the tampon circumference. For example, a groove may extend over at least about 10°, 20°, 30°, 40°, 50°, 60°, 70°, 80°, 90°, 100°, 120°, 130°, 140° or 150°. A tampon may comprise varying number (even or odd) of such grooves and ribs. The number of grooves and ribs may depend on the diameter of the tampon. The number of grooves and ribs may also depend on the type of absorbent material included within the absorbent core of the tampon. Preferably, a tampon may comprise between 3 and 12 grooves and/or ribs, *e*.*g*., 3, 4, 5, 6, 7, 8, 9, 10, 11 or 12, more preferably between 6 and 12, and even more preferably at least about 8 grooves or ribs. Preferably, the longitudinal grooves may be spaced at regular intervals.

In another embodiment, the height of the withdrawal end may be smaller than 50% of the height of the tampon. Preferably, the height of the withdrawal end may be smaller than 40% of the height of the tampon. More preferably the height of the withdrawal end may range from 1 to 30% of the height of the tampon. Even more preferably the height of the withdrawal end may be 1, 5, 10, 15, 20, 25, 30% of the height of the tampon, or a value in the range between any two of the aforementioned values.

In another embodiment, after use, the diameter of the withdrawal end may be smaller than the average diameter of the tampon. In a preferred embodiment, after use, the diameter of the withdrawal end may be at least smaller than 80% of the average diameter of the tampon and preferably may be at least smaller than 50% of the average diameter of the tampon. Preferably, after use, the diameter of the withdrawal end may range from 5 to 50% of the average diameter of the tampon and more preferably may range from 10 to 50% of the average diameter of the tampon. In particular, the diameter of the withdrawal end may be, after use, 10, 15, 20, 25, 30, 35, 40, 45 or 50% of the average diameter of the tampon, or a value in the range between any two of the aforementioned values.

In another embodiment, the diameter-restraining element may be a hydrophobic or liquid impervious element. The hydrophobic or liquid impervious element may be made of a hydrophobic or liquid impervious material. As used herein, the term "hydrophobic" refers to an element or a material which is not permeable to hydrophilic liquid and which has a contact angle of at least 90°. Preferably, the hydrophobic or liquid impervious material may be a thermoplastic material. By means of preference, but not of limitation, the hydrophobic material may be polyethylene, polypropylene, polyethylene terephthalate, polyester, polystyrene, polyamide, polyether, polyurethane, copolymers or blends thereof. Preferred thermoplastic polymeric materials may include polyethylene or polypropylene, copolymers or blends thereof, preferably polyethylene. More preferably, the diameter-restraining element may be a liquid-impervious element made of a liquid-impervious material.

In a preferred embodiment, the height of the diameter-restraining element may be at least 1% of the height of the tampon. In a preferred embodiment, the height of the diameter-restraining element may range from 1 to 50% of the height of the tampon. Preferably, the height of the diameter-restraining element may range from 1 to 30% of the height of the tampon and more preferably may range from 5 to 30% of the height of the tampon. In yet another preferred embodiment, the height of the diameter-restraining element may be 5, 6, 7, 8, 9, 10, 11, 12, 13, 14, 15, 16, 17, 18, 19, 20, 21, 22, 23, 24, 25, 26, 27, 28, 29 or 30 % of the height of the tampon, or a value in the range between any two of the aforementioned values.

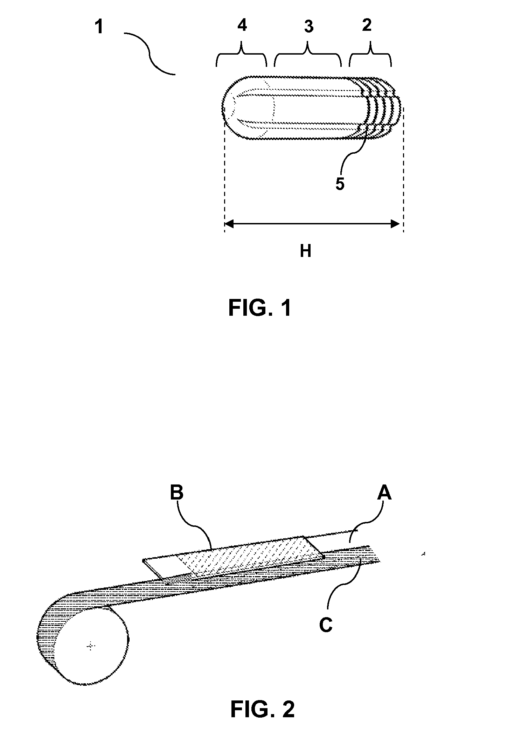

With reference to FIG.1, a tampon according to an embodiment of the invention is illustrated. The tampon **1** comprises an insertion end **4** and a withdrawal end **2** and a main portion **3.** The tampon **1** is provided with a diameter restraining element **5** at the withdrawal end **2.** The outer surface, the inner surface (not shown) and/or the bottom circle (not shown) of the withdrawal end **2** may be provided with said diameter restraining element **5.** The height H represents the height of the tampon.

In a preferred embodiment, the diameter-restraining element may cover the bottom circle of the withdrawal end and at least part of the withdrawal end outer surface. In a preferred embodiment, the diameter-restraining element may cover the bottom circle of the withdrawal end and at least 50% of the outer surface of the withdrawal end. Preferably, the diameter restraining element may cover the bottom circle of the withdrawal end and from 60% to 100% of the withdrawal end outer surface. More preferably, the diameter-restraining element may cover the bottom circle of the withdrawal end and 60, 65, 70, 75, 80, 85, 90, 95 or 100% of the withdrawal end outer surface, or a value in the range between any two of the aforementioned values.

In another embodiment, said diameter-restraining element may comprise at least one continuous or discontinuous strip or ring of a hydrophobic or liquid impervious material. If more than one continuous or discontinuous strips or rings are used, they can be superimposed or juxtaposed one other. In a preferred embodiment, said continuous or discontinuous strip or ring is configured to restrain the diameter of the tampon and to avoid leakage at the withdrawal end of the tampon.

In a preferred embodiment, the diameter-restraining element may comprise at least one continuous strip or ring of a hydrophobic or liquid impervious material. The continuous strip or ring may have a length lower, equal or greater than the outside perimeter of the tampon. The continuous strip or ring may have any shape.

In a preferred embodiment, the length of the continuous strip or ring may be at least 50% of the outside perimeter of the tampon. Preferably the length of the continuous strip or ring may range from 75% to 500% of the outside perimeter of the tampon; more preferably may range from 75% to 250%. Preferably, the length of the continuous strip or ring may be 75, 80, 85, 90, 95, 100, 105, 110, 115, 120, 125, 130, 135, 140, 145, or 150% of the outside perimeter of the tampon, or a value in the range between any two of the aforementioned values.

In another preferred embodiment, the diameter-restraining element may comprise from 1 to 50 continuous strips or rings. Preferably the diameter restraining element may comprise from 1 to 30 continuous strips or rings, more preferably from 1 to 25 continuous strips or rings. Even more preferably the diameter restraining element may comprise 1, 2, 3, 4, 5, 6, 7, 8, 9, 10, 11, 12, 13, 14, 15, 16, 17, 18, 19, 20, 21, 22, 23, 24 or 25 continuous strips or rings.

Alternatively, the diameter restraining element may comprise discontinuous strips or rings of the hydrophobic or liquid-impervious material. The term "discontinuous strips or rings" as used herein refers to an assembly of small continuous strips or rings. In a preferred embodiment, the discontinuous strips or rings may comprise at least two small continuous strips or rings. Preferably the discontinuous strips or rings may comprise from 2 to 100 small continuous strips or rings, more preferably the discontinuous strips or rings may comprise between 2 and 50 small continuous strips or rings. Even more preferably, the discontinuous strips or rings may comprise 2, 5, 10, 15, 20, 25, 30, 35, 40, 45 or 50 small continuous strips or rings, or a value in the range between any two of the aforementioned values. The length of the small continuous strips or rings may be at least smaller than 50% of the outside perimeter of the tampon. Preferably, the length of the small continuous strips or rings may range from 5% to 50% of the outside perimeter of the tampon.

When a tampon is provided with recessed or raised portions (e.g. grooves or ribs...) the diameter-restraining element may partly or wholly cover said portions. Alternatively, when a tampon is provided with recessed or raised portions (e.g. grooves or ribs...) the diameter restraining element may not cover said portions.

In another embodiment, the outer surface of said diameter-restraining element may be provided with at least one identification element. Alternatively, the outer surface of the tampon may be provided with at least one identification element. Alternatively, the outer surface of said diameter-restraining element and of said tampon may be provided with at least one identification element. As used herein, the term "identification element" refers to a signal perceptible by the user that corresponds to a functionally distinguishable characteristic of a feminine hygiene article. The functionally distinguishable characteristic of a feminine hygiene article is a characteristic that is different in surface area from other products in the same array. The distinguishable characteristic may be, e.g., the absorption capacity, the expansion capacity of the tampon or of part of it. The identification element may include words, graphics, alphabetic letters or a geometric form identifying the product in use. For example, the identification element may be a circle, a polygon, a logo, a trademark. Alternatively, the diameter-restraining element in itself may act as an identification element. In such case, the colour, size, shape, material, surface structure of the diameter-restraining element may be a signal perceptible by the user and corresponding to a functionally distinguishable characteristic of the tampon.

In a preferred embodiment, said identification element may be stamped, impregnated, embossed, printed, folded or coated on the outer surface of the diameter-restraining element. Preferably, the identification element may be printed or coated on the outer surface of the diameter-restraining element. Alternatively, the identification element may be printed or coated on the outer surface of the tampon. When the tampon is provided with an outer cover, the identification element may be printed or coated on the outer cover surface. Various printing methods may be used including letterpress, flexography, gravure, offset lithography, screen and inkjet.

In particular, the diameter-restraining element may comprise one continuous strip of a hydrophobic material and may have a height of 5 to 20% of the length of the tampon.

In another embodiment, the tampon may be provided with an outer cover. The outer cover may be made of a non-woven material, a woven material or an apertured polymeric film. As used herein, the term "non-woven" refers to a class of fabrics comprising fibres or threads assembled into web. The materials are processed into isotropic fabrics by arranging the fibres more or less randomly. The fibres in non-woven materials are interlaid but not in a regular or identifiable manner.

As used herein, the term "woven" refers to a class of fabrics comprising fibres, threads or yarns assembled into web by interlaying in a regular or identifiable manner. The term refers to any woven material such as a woven textile, web, mesh, screen, etc.
The apertured polymeric film may be a thermoplastic material. By means of preference, but not of limitation, the thermoplastic material may be polyethylene, polypropylene, polyethylene terephthalate, polyester, polystyrene, polyamide, polyether, polyurethane, copolymers or blends thereof. Preferred thermoplastic material may include polyethylene, polypropylene, copolymers or blends thereof. Alternatively, the apertured polymeric film may be made of an elastic material. The terms "elastic" describes the ability of materials to undergo deformation in at least one direction when a force is applied to them and to resume substantially to their original dimensions upon relaxing. The elastic elongation of the apertured outer cover may range from 5% to 700%. Preferably, the elastic elongation of the apertured outer cover may be about 10%, 20%, 30%, 40% 50%, 100%, 150%, 200%, 250%, 300%, 400% or 500%. The elastic material may be polyisoprene, butadienestyrene copolymers, styrene block copolymers (*e*.*g*., styrene/isoprene/styrene (SIS), styrene/butadiene/styrene (SBS), or styrene/ethylene-butadiene/styrene (SEBS) block copolymers), olefinic elastomers, polyether esters, polyurethanes. In addition, other additives can be added to the elastic or thermoplastic material *e*.*g*. antioxidants, lubricants, antiblock and antislip agents, plasticizers, nucleating agents, antistatic agents, flame retardants, pigments, dyes, and inorganic or organic fillers.

In a preferred embodiment, the diameter-restraining element may be disposed underneath the outer cover of the tampon. Alternatively, the diameter-restraining element may be disposed above the outer cover.

In another aspect of the invention, a method for manufacturing a tampon according to the invention is provided. In a preferred embodiment, the method may comprise the step of providing a diameter-restraining element at the withdrawal end of the tampon.

In another preferred embodiment, the method may comprise the steps of:
- providing a diameter-restraining element at the withdrawal end of a tampon blank,
- inserting the tampon blank in a tampon pressing section of a press, and
- pressing essentially radially the tampon blank in the press jaws to form the tampon,
   or
- providing a tampon blank and inserting said tampon blank in a tampon pressing section of a press,
- pressing essentially radially the tampon blank in the press jaws to form the tampon, and
- providing the diameter-restraining element at the withdrawal end.
The method may further comprise the step of providing an identification element on the outer surface of said diameter restraining element.

The method can be performed to manufacture a so-called "swiss-roll" or width-wise expanding tampon. The method can also be performed to manufacture so-called W-folded or width- and length-wise expanding tampons.

In a preferred embodiment, a method for manufacturing a swiss-roll tampon is provided. Said method for manufacturing a swiss-roll tampon may comprise the steps of:
a) providing a ribbon of absorbent core and the diameter-restraining element, wherein the width of said ribbon of absorbent core corresponds approximately to the length of the tampon,
b) positioning said diameter-restraining element above a portion of said ribbon of the absorbent core,
c) folding the diameter-restraining element around the absorbent core ribbon by means of a folding plate,
d) rolling up the material provided in step c) about an axis that is located transverse to the longitudinal direction of said material and outside the longitudinal centre of said material to form a tampon blank,
e) inserting the tampon blank in a tampon pressing section of a press for manufacturing a tampon,
f) pressing essentially radially the tampon blank in the press jaws to form the tampon.

In one embodiment, the method may further comprise the step of printing or coating at least one identification element on the outer surface of said diameter-restraining element. In another embodiment, the method may further comprise the step of printing or coating at least one identification element on the outer surface or on the outer cover of the tampon.

In another embodiment, the method may further comprise the step of providing a ribbon of a non-woven material which forms the outer cover of the tampon. The method may further comprise the step of positioning the ribbon of diameter-restraining material underneath the ribbon of said non-woven material. Alternatively, the method may further comprise the step of positioning the ribbon of diameter-restraining material above the ribbon of said non-woven material.

The method of manufacturing a "swiss-roll" tampon provided with a diameter-restraining element is now described. It is obvious that the same principles can also be applied to the so-called W-folded or width- and length-wise expanding tampons.

With reference to FIG. 2, one step in a process for producing a swiss-roll tampon according to an embodiment of the invention is illustrated. A ribbon of an elongated absorbent core **A,** a ribbon of a diameter-restraining element **C** and a ribbon of a non-woven material **B** is provided. The ribbon **A** has a width which corresponds approximately to the length of the tampon. The ribbon of the diameter-restraining element **C** is positioned above a portion of the ribbon **A** and underneath the ribbon **B.** The ribbon **C** can be sealed to the ribbon **A** whereas the ribbon **B** can be simultaneous sealed to both ribbons **A** and **C** using heat and pressure. The length of the ribbon **C** can be approximately equal or longer than the length of the ribbon **A.** In this case, the ribbon **C** may be configured to act as a diameter-restraining element and an anti-leakage element. Alternatively, the length of the ribbon **C** can be shorter than the length of the ribbon **A** and thus may be only configured as a diameter-restraining element. The length of the ribbon **B** can be smaller than the length of the ribbon **A,** but is longer than the circumference of the tampon blank. The ribbon **B** forms the outer cover of the tampon and has a smooth surface. Said ribbon **B** serves the purpose of making the insertion of the tampon into the bodily cavity significantly easier, even outside the time of heavier menstruation. The ribbon **A** partly covered with ribbons **B** and **C** can be disposed within a winding mandrel (not shown). A withdrawal cord (not shown) can lay around the ribbon **A** and the winding mandrel, transversely to the longitudinal direction of the ribbon **A,** and, if necessary, subsequently knotted at its free end.

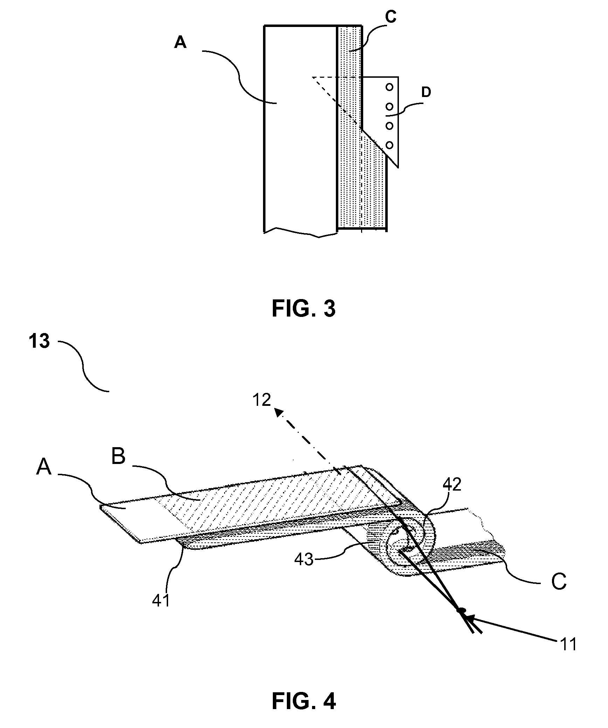

With reference to FIG. 3, the folding step of the diameter-restraining element around the elongated absorbent core according to an embodiment of the invention is illustrated. The ribbon of said diameter-restraining material **C** is disposed above a portion of said ribbon **A** of the elongated absorbent core. The ribbon **C** is folded around a portion of the ribbon **A,** by means of a folding plate **D.**

With reference to FIG. 4, the manufacturing of the tampon blank **13** according to an embodiment of the invention is illustrated. The ribbon **A,** partly covered with ribbons **B** and **C,** is cut to a defined length. The length, the thickness and the density of the ribbon **A** determine the mass of the tampon and are chosen in function of the desired absorption capacity. The ribbon **A** is then rolled up on itself about an axis **12** that is located transverse to the longitudinal direction of said ribbon **A** and outside the longitudinal centre of said ribbon **A.** For instance, the axis **12** goes through the longitudinal axis of the tampon blank **13.** A uniform material distribution on the outer surface of the tampon blank **13** is achieved. The diameter-restraining element covers the outer surface **41,** the inner surface **43** and the bottom circle 42 of the withdrawal end. The tampon blank **13** may be essentially cylindrical.

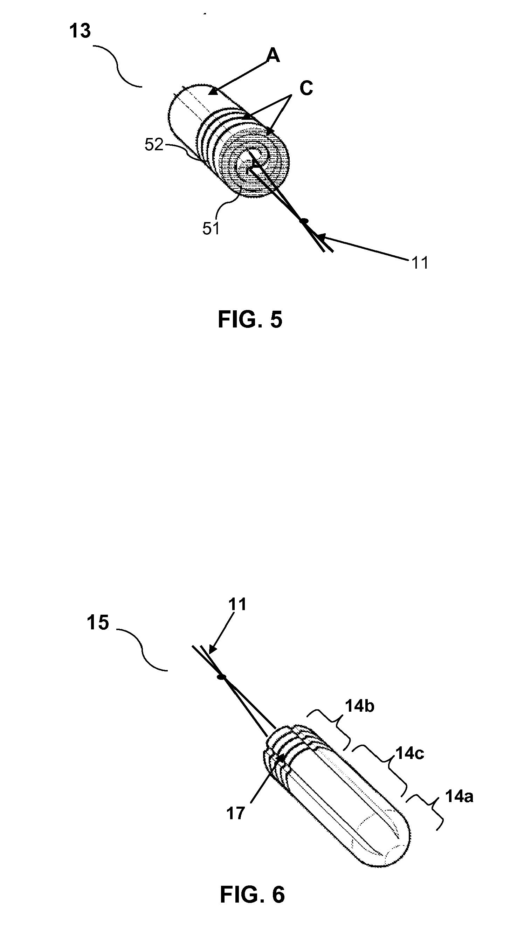

With reference to FIG. 5, a tampon blank according to an embodiment of the invention is illustrated. The tampon blank **13** comprises an elongated absorbent core **A,** a strip of the diameter-restraining element **C** and an outer cover made of a non-woven material (not shown). The tampon blank further comprises a withdrawal cord **11.** The tampon blank **13** is illustrated, prior to being inserted in the tampon pressing section of a tampon machine. The diameter-restraining element **C** covers a portion of the outer surface **52,** the inner surface (not shown) and the bottom circle **51** of the withdrawal end. The tampon blank **13,** finished by being wound up into a cylindrical shape, is then fed to a press. Said tampon blank **13** is pressed essentially radially into the final shape of the tampon **15** (see FIG. 6).

With reference to FIG. 6, a tampon **15** after leaving the tampon pressing section according to an embodiment of the invention is illustrated. The tampon **15** comprises a withdrawal cord **11,** an insertion end **14a,** a withdrawal end **14b** and longitudinal main portion **14c.** The withdrawal end **14b** is provided with at least one continuous strip of a hydrophobic material **17.** The diameter of the withdrawal end **14b** is approximately equal to the average diameter of the tampon.

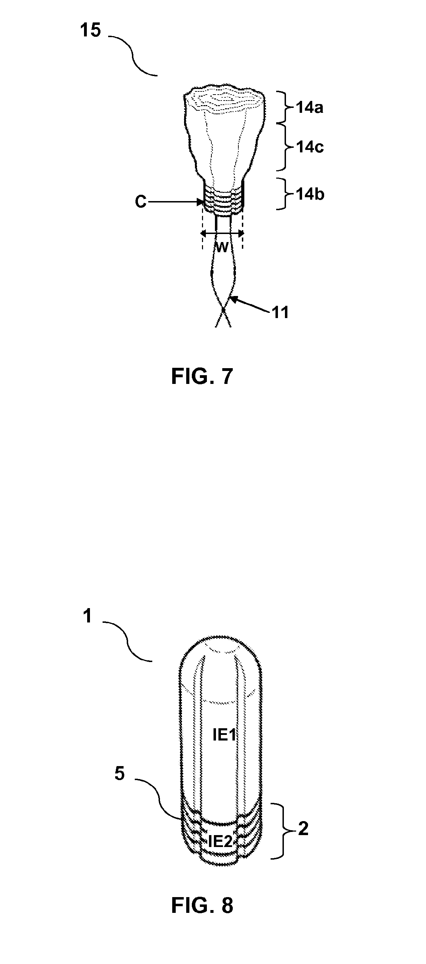

With reference to FIG. 7, a tampon **15** according to an embodiment of the invention is illustrated, after use. The tampon **15** comprises a withdrawal cord **11,** an insertion end **14a,** a withdrawal end **14b** and longitudinal main portion **14c.** The withdrawal end **14b** is provided with the diameter-restraining element **C.** The diameter of the withdrawal end **14b** is lower than the average diameter of the tampon. The withdrawal end provided with the diameter-restraining element has low extension capacities. This results in an easy removal of the tampon after use.

With reference to FIG. 8, a tampon **1** according to an embodiment of the invention is illustrated. The tampon **1** comprises a diameter-restraining element **5** at the withdrawal end **2.** Two identification elements **IE1** and **IE2** are respectively provided on the outer surface of the tampon and on the outer surface of the diameter-restraining element **5.** The identification elements can be words, graphics, logo or trademark. The identification **IE2** may be absent and, thus, the diameter-restraining element is in itself an identification element.

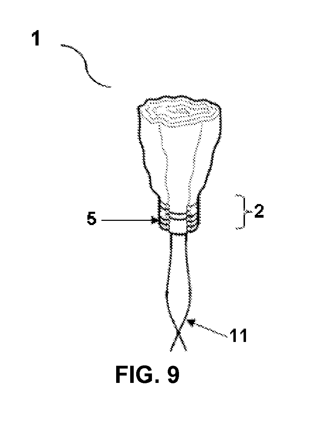

With reference to FIG. 9, a tampon **1,** after use, according to an embodiment of the invention is illustrated. The tampon **1** comprises a diameter-restraining element **5** at the withdrawal end **2.** The diameter-restraining element is made of a plurality of discontinuous strips. The diameter of the withdrawal end is smaller than the average diameter of the tampon.

The tampon according to the invention is suitable for capturing body fluids. The teachings of the invention may be applied to digital tampons or tampons with an applicator. The term "digital tampon" refers to a tampon which is intended to be inserted into the vaginal canal, with the user's finger and without the aid of an applicator. When the tampons are intended to be digitally inserted, they may be provided with a finger indent or recess at the withdrawal end of the tampon to aid their insertion. The term "applicator" may be a telescoping-tube type applicator, including the tube-and-plunger type or the compact type arrangements. The applicator may be plastic, paper, or any other suitable material, *e*.*g*., may be moulded polyethylene.

In another aspect, the invention relates to an apparatus specifically configured for producing or manufacturing the tampon as disclosed herein.

## Claims

1. Tampon, particularly for feminine hygiene, comprising an elongated absorbent core having an insertion end, a withdrawal end, **characterised in that** said tampon is provided with a diameter-restraining element at the withdrawal end.

2. Tampon according to claim 1, wherein after use, the diameter of the withdrawal end is smaller than the average diameter of the tampon.

3. Tampon according to claims 1 or 2, wherein after use, the diameter of the withdrawal end is at least smaller than 80% of the average diameter of the tampon.

4. Tampon according to any of previous claims 1 to 3, wherein the diameter-restraining element covers the bottom circle and at least part of the withdrawal end outer surface.

5. Tampon according to any of previous claims 1 to 4, wherein the diameter-restraining element covers the bottom circle of the withdrawal end and at least 50% of the withdrawal end outer surface.

6. Tampon according to any of previous claims 1 to 5, wherein said diameter-restraining element is a hydrophobic or liquid impervious element.

7. Tampon according to any of previous claims 1 to 6, wherein the outer surface of said diameter-restraining element is provided with at least one identification element.

8. Tampon according to any of previous claims 1 to 7, wherein the diameter-restraining element comprises at least one continuous or discontinuous strip or ring of a hydrophobic material.

9. Tampon according to claim 8, wherein said continuous or discontinuous strip or ring is configured to restrain the diameter of the tampon and to avoid leakage at the withdrawal end of the tampon.

10. Tampon according to any of previous claims 1 to 9 wherein the tampon is provided with a non-woven material which forms the outer cover of said tampon.

11. A method for manufacturing a tampon according to any of previous claims 1 to 10, comprising the steps of:
- providing a diameter-restraining element at the withdrawal end of a tampon blank,
- inserting the tampon blank in a tampon pressing section of a press, and
- pressing essentially radially the tampon blank in the press jaws to form the tampon,
or
- providing a tampon blank and inserting said tampon blank in a tampon pressing section of a press,
- pressing essentially radially the tampon blank in the press jaws to form the tampon, and
- providing a diameter-restraining element at the withdrawal end.

12. A method for manufacturing a swiss-roll tampon according to any of previous claims 1 to 10, comprising the steps of:
a) providing a ribbon of an absorbent core and a diameter-restraining element, wherein the width of said ribbon of the absorbent core corresponds to the length of the tampon,
b) positioning said diameter-restraining element above a portion of said ribbon of the absorbent core,
c) folding the diameter-restraining element around the ribbon of the absorbent core by means of a folding plate,
d) rolling up the material provided in step c) about an axis that is located transverse to the longitudinal direction of said material and outside the longitudinal centre of said material to form a tampon blank,
e) inserting the tampon blank in a tampon pressing section of a press for manufacturing a tampon,
f) pressing essentially radially the tampon blank in the press jaws to form the tampon.

13. Method according to claim 11 or 12, further comprising the step of providing at least one identification element on the outer surface of said diameter-restraining element.

14. Apparatus specifically configured for producing or manufacturing the tampon according to any of previous claims 1 to 10.
